(19) Europäisches Patentamt
European Patent Office
Office européen des brevets

(11) EP 1 436 412 B1

# (12) FASCICULE DE BREVET EUROPEEN

(45) Date de publication et mention de la délivrance du brevet:
**12.07.2006 Bulletin 2006/28**

(21) Numéro de dépôt: **02783229.4**

(22) Date de dépôt: **03.10.2002**

(51) Int Cl.:
***C12Q 1/64*** *(2006.01)*

(86) Numéro de dépôt international:
**PCT/FR2002/003387**

(87) Numéro de publication internationale:
**WO 2003/031644 (17.04.2003 Gazette 2003/16)**

(54) **METHODE POUR MODELISER LA BIODEGRADATION D'HYDROCARBURES DANS UN GISEMENT PETROLIER**

VERFAHREN ZUR MODELLIERUNG DEN BIOABBAU DER KOHLENWASSERSTOFFEN IN EINES ÖLFELD

METHOD FOR MODELLING HYDROCARBON DEGRADATION IN AN OIL DEPOSIT

(84) Etats contractants désignés:
**ES GB IT NL**

(30) Priorité: **05.10.2001 FR 0112892**

(43) Date de publication de la demande:
**14.07.2004 Bulletin 2004/29**

(73) Titulaire: **Institut Français du Pétrole**
**92852 Rueil-Malmaison Cedex (FR)**

(72) Inventeurs:
- **CARPENTIER, Bernard**
  **F-95690 Labbeville (FR)**
- **MARTIN, Ludovic**
  **F-93000 Bobigny (FR)**

(56) Documents cités:
- **LU G ET AL: "NATURAL ATTENUATION OF BTEX COMPOUNDS: MODEL DEVELOPMENT AND FIELD-SCALE APPLICATION" GROUND WATER, TER WELL JOURNAL PUB. CO, WORTHINGTON, OH, WA, vol. 37, no. 5, septembre 1999 (1999-09), pages 707-717, XP001148707 ISSN: 0017-467X**
- **COZZARELLI ISABELLE M ET AL: "Geochemical heterogeneity of a gasoline-contaminated aquifer." JOURNAL OF CONTAMINANT HYDROLOGY, vol. 40, no. 3, 15 décembre 1999 (1999-12-15), pages 261-284, XP002214456 ISSN: 0169-7722**
- **PECHER K ET AL: "REDUKTIVE DEHALOGENIERUNG VON CHLORKOHLENWASSERSTOFFEN WAEHREND DER ANAEROBEN STABILISIERUNG VON HAUSMUELL REDUCTIVE DEHALOGENATION OF CHLORINATED HYDROCARBONS DURING ANAEROBIC STABILIZATION OF MUNICIPAL WASTES" ACTA HYDROCHIMICA ET HYDROBIOLOGICA, VCH, WEINHEIM, DE, vol. 23, no. 6, 1995, pages 271-279, XP008013271 ISSN: 0323-4320**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Désignation du domaine technique

La présente invention concerne une méthode pour modéliser la biodégradation d'hydrocarbures piégés dans un gisement pétrolier ou piège, par action de la population bactérienne dans un aquifère, à partir de données relatives au réservoir étudié.

La méthode selon l'invention permet de former un outil d'évaluation très utile notamment aux géologues soucieux d'orienter les investigations hors de zones à risque.

### Etat de la technique

La biodégradation d'une huile est un phénomène d'altération provoquée par l'oxydation de certaines molécules hydrocarbonées par des micro-organismes ou flore bactérienne, qui conduit à la formation d'une huile lourde donc difficile à produire et commercialement peu rentable. Les bactéries consomment ces molécules dans le cadre de leur respiration et pour se procurer les éléments indispensables à leur croissance et leur réplication. L'étude de ce phénomène suscite un regain d'intérêt avec le développement de l'exploration dans les grands fonds où la présence d'huile lourde est un des risques majeurs. Il existe actuellement peu de moyens pour prédire les risques de biodégradation et pour la décrire alors que la nécessité économique de développement d'outils quantitatifs est forte aujourd'hui.

La biodégradation est un processus bio-géochimique qui s'apparente à une combustion froide opérée par des micro-organismes. Une bactérie capable de dégrader des composés hydrocarbonés peut être en effet considérée comme une machine à consumer des hydrocarbures, à l'aide d'ions accepteurs d'électrons (pouvant être comparer à un comburant) et à rejeter un réducteur.

Une première condition à l'existence de la biodégradation est naturellement l'existence de ces micro-organismes. Elles sont présentes dans le milieu, soit depuis le dépôt de la couche de sédiments en surface soit parce qu'elles ont été apportées par des eaux météoriques. En l'absence de matière organique pétrolière ou d'autres sources de carbone (CO2, ions carbonatés, etc.) les bactéries s'enkystent et peuvent être préservées pendant des périodes de temps extrêmement longues.

Il existe, on le sait, deux mécanismes bactériens distincts entraînant la dégradation de matière organique.

- Le *catabolisme* appelé aussi respiration. C'est le processus de décomposition de la matière organique par oxydation en vue de fournir de l'énergie stockée dans les molécules ATP (Adénosine TriPhosphate). Il nécessite matière organique et accepteur d'électron (pas nécessairement de l'oxygène). Le bilan chimique est encore mal connu et change pour chaque molécule hydrocarbonée.

- *L'anabolisme.* C'est le processus de formation de matière cellulaire permettant la réplication et la croissance bactérienne. La bactérie a besoin de tous les éléments qui la constituent, C, O, N, S, K, P principalement. La connaissance des proportions de chacun de ces éléments dans une bactérie fournirait un premier bilan de leur consommation relative dans le milieu. L'anabolisme utilise l'énergie apportée par la respiration pour effectuer ses réactions chimiques. Le bilan chimique global d'une bactérie effectuant son anabolisme est donc une combinaison de celui lié à la création de matière cellulaire et de la respiration.

La respiration est un processus permanent tandis que l'anabolisme n'a lieu qu'à certains moments de la vie de la bactérie. Seule la respiration a été étudiée car on l'estime prépondérante sur l'anabolisme dans la dégradation des hydrocarbures. Les deux mécanismes ne sont pas indépendants cependant. Lorsqu'une bactérie réalise sont processus anabolique elle accroît sa respiration car elle a besoin de beaucoup d'énergie.

Le tableau ci-après donne des exemples de sources et accepteurs d'électrons ainsi que des produits des réactions que l'on peut rencontre dans les réservoirs pétroliers.

| Accepteurs d'électrons | Sources d'électrons | produits |
|---|---|---|
| $O_2$ | HC | $CO_2$ |
| $MnO_2$ | HC,H+ | $CO_2$, $Mn^{2+}$ |
| $NO^-_3$ | HC, H+ | $CO_2$, $N_2$ |
| $Fe(OH)_3$ | HC, H+ | $CO_2$, $Fe^{2+}$ |

(suite)

| Accepteurs d'électrons | Sources d'électrons | produits |
|---|---|---|
| $SO_4^{2-}$ | HC, HC+ | $CO_2$, $H_2S$ |
| $CO_2$ éventuel | HC | $CO_2$, $CH_4$ |

Un modèle connu de biodégradation d'un champ à partir de données issues du champ de Gullfaks en mer du Nord, est décrit dans la publication suivante :

- Horstad I., Larter S.R., Mills N., A quantitative model of biological petroleum degradation within the Brent group reservoir, Org. Geochem., 19, pp 107-117.

Suivant ce modèle on envisage le remplissage d'un piège en hydrocarbures avec un flux constant. De l'eau saturée en accepteurs d'électrons circule également à flux constant. Le champ a une symétrie parallélépipédique simple. Au cours du remplissage dans la zone de transition, la destruction de quatre n-alcanes est calculée à l'aide de lois cinétiques classiques du premier ordre obtenues en laboratoire. Le bilan équationnel est constitué d'un terme cinétique de destruction d'hydrocarbure et les termes d'approvisionnement en hydrocarbure et en accepteurs d'électrons par convection. La dégradation est double, aérobie et par sulfato-réduction.

Dans ce système, l'alimentation en accepteurs d'électrons est le facteur limitant. Les paramètres contrôlant le système sont l'épaisseur de la zone de transition, le débit d'eau sous la zone de transition. Les résultats obtenus par ce type de modèle se révèlent peu réalistes. Cela tient au choix des bilans et des cinétiques de réaction, celles-ci étant liées à la méconnaissance de la cinétique bactérienne et les mécanismes d'attaque développés par les bactéries.

Des modèles intégrant une approche plus complexe du milieu poreux et du transport de matière sont couramment utilisés pour simuler la biodégradation dans les nappes polluées peu profondes. Il s'agit notamment du modèle SIMUSCOP qui permet de mailler en 2D un sous-sol et de calculer la biodégradation aérobie sur les BTEX, que le demandeur a développé, sur la base de travaux décrits dans la référence suivante :

- Côme, J.M. Expérimentation et modélisation de procédés in situ de dépollution par biodégradation aérobie des aquifères contaminés par des hydrocarbures, mémoire de thèse, pp 75-93, avril 95.

On peut citer également le logiciel BIO1D développé par la société ECHOSCAN, RT3D ou encore PARSSIM1 (Université du Texas). De la documentation concernant ces modèles est disponible aux adresses Internet suivantes :

- Modèle BIO1D : http://people.becon.org/~echoscan/13-22.htm
- Modèle PARSSIM : http://king.ticam.utexas.edu/Groups/SubSurfMod/ColorPictures/caption.html
- Modèle RT3D : http://bioprocess.pnl.zov/rt3d descrip.htm.

Une bibliographie concernant la simulation de biodégradation dans le cadre de la dépollution est également disponible à l'adresse :

- http://www.nal.usda.gov/wqic/Bibliographies/qb9406.html.

Dans la plupart de ces modèles, on ne s'intéresse qu'aux molécules hydrocarbonées présentant une forte solubilité dans l'eau (BTEX). L'huile y est donc présente sous forme dissoute et ne se déplace que par diffusion. Parfois de l'huile résiduelle se déplaçant par convection est prise en considération également. Bien que les saturations d'huiles mises en jeu ne sont pas les mêmes que dans un réservoir pétrolier et que l'accent est mis sur les transports de matière dans l'aquifère, la problématique mathématique est au fond applicable aux réservoirs.

Les équations mises en place dans tous ces modèles sont de la forme suivante :

$$\frac{\partial c\alpha}{\partial t} = \frac{\partial}{\partial xi}\left( Vc\alpha - D\frac{\partial c\alpha}{\partial xi} \right) + q\alpha c\alpha \qquad (1)$$

Dans cette équation où $\frac{\partial c\alpha}{\partial t}$ est un terme d'accumulation, $Vc_\alpha$ est un terme de transport, $D\frac{\partial c_\alpha}{\partial xi}$ est un terme de réaction du 1er ordre et $q_\alpha c_\alpha$ est un terme source,

T est letemps

xi est une variable spatiale en x, y et z

P est le nombre d'espèces chimiques

V(xi,t) est le champ de vitesses d'un fluide (l'eau).

D(xi,t) est le coefficient de diffusion,

$C_\alpha$ est la concentration de l'espèce $\alpha$, et

$q_\alpha$ est le coefficient cinétique de réaction du premier ordre de l'espèce $\alpha$.

La fermeture du problème est assurée à l'aide d'un certain nombre de conditions initiales et de conditions limites telles que les concentrations initiales, les zones sources de diffusion, les zones de transport impossible, etc.

Pour décrire un milieu poreux géologique, des modèles en 3D ont été développés dans lesquels on maille une zone d'aquifère et on détermine au sein de chaque maille, les champs de vitesse et les concentrations.

Tous ces modèles offrent une description très réaliste du milieu géologique mais ils ne considèrent que des huiles de composition peu élaborée, se limitant à quelques molécules parmi les plus solubles ou même à une seule molécule hydrocarbonée type. Ces modèles ne sont donc pas utilisables en soi pour modéliser la biodégradation dans les réservoirs en vue d'obtenir une description de l'évolution de l'huile. En outre, pour une application à des échelles de temps géologiques, le problème demeure le type de cinétique appliquée pour les réactions de biodégradation.

**La méthode selon l'invention**

La méthode selon l'invention permet de modéliser la biodégradation progressive d'hydrocarbures piégés dans un réservoir pétrolier ou piège étudié, par action d'une population bactérienne dans un aquifère, à partir de données relatives au réservoir étudié, portant sur la forme et la hauteur du réservoir, les caractéristiques physiques du milieu poreux, l'épaisseur de la zone de transition entre les hydrocarbures et l'eau, la composition des hydrocarbures, du flux d'accepteurs d'électrons rentrant dans le réservoir et de données sur la population bactérienne dans l'aquifère, dans le but de déterminer les conditions d'exploitation du réservoir.

Elle est caractérisée en ce qu'elle comporte :

- la discrétisation du réservoir par un maillage dans lequel chaque maille a pour hauteur l'épaisseur de la zone de transition ; et

- la détermination de la variation sur la hauteur du dit réservoir, de la proportion en fractions lourdes des hydrocarbures sous l'effet de la biodégradation par ajustement progressif au sein de chaque maille de la population bactérienne à la quantité d'hydrocarbures disponible, à l'espace poreux disponible, à la quantité d'accepteurs d'électrons présente dans le réservoir et des capacités de dégradation de la dite population.

Suivant un mode de mise en oeuvre, on détermine au préalable le taux de remplissage initial du réservoir en hydrocarbures quand les conditions de température régnant dans le réservoir se prêtent à la biodégradation.

En prenant en compte les effets à long terme de la biodégradation des huiles d'un gisement, la méthode permet de faire des estimations beaucoup plus réalistes qu'avec les méthodes antérieures, de la répartition des fractions composant les hydrocarbures et de mieux sélectionner les zones d'exploitation du gisement.

**Présentation sommaire des figures**

D'autres caractéristiques et avantages de la méthode selon l'invention, apparaîtront à la lecture de la description ci-après d'exemples non limitatifs de mise en oeuvre, en se référant aux dessins annexés où :

- la Fig.1a montre schématiquement, dans un piège géologique en cours de remplissage, le déplacement d'une zone de transition eau/hydrocarbure, la biodégradation ayant lieu dans la zone de transition qui se déplace vers le bas au fur et à mesure que le champ se remplit, cas où l'ensemble de l'huile peut être totalement biodégradée ;

- la Fig.1b montre schématiquement, une autre situation où la biodégradation n'intervient qu'après remplissage du piège, dans la zone de transition eau/hydrocarbure située à la base du gisement ; la biodégradation "remonte" lentement dans le réservoir et n'affecte que la partie basale du réservoir ;

- la Fig.2 montre schématiquement la zone de transition eau/hydrocarbure à l'intérieur d'une maille du piège discrétisé par un maillage ;

- les Fig.3a, 3b montrent respectivement de façon schématique, un milieu poreux "réel" et le milieu poreux simplifié où, pour le calcul de la surface de contact eau/huile à l'échelle du pore, l'ensemble de l'huile présente dans le pore est représenté par une sphère unique dont le volume respecte la saturation en huile du pore ;

- la Fig.4 montre un exemple d'évolution de population bactérienne calculée par le modèle en fonction des tailles de pore dans un milieu poreux contenant de l'huile à différentes saturations ;

- la Fig.5 montre un exemple d'organigramme logique du calcul de la population bactérienne, qui est ajustée de manière à être compatible avec les paramètres pétrophysiques (volume poreux, saturation...) et la quantité d'accepteurs d'électrons présente dans la maille à un temps T ;

- la Fig.6 montre un exemple de résultat d'un calcul réalisé avec le modèle dans le scénario 2 où la biodégradation a lieu en cours de remplissage); la variation de la composition de l'huile sur la verticale du champ, liée à la géométrie du piège est clairement visible;

- la Fig.7 montre les résultats d'une simulation de biodégradation avec vérification de la simulation par rapport aux compositions mesurées sur un cas réel (Amérique du Sud - Huile biodégradée 1 et 2) ;

- la Fig.8 montre un organigramme logique du calcul de la biodégradation sur une verticale d'un piège géologique suivant un premier scenario de biodégradation ; et

- la Fig.9 montre un organigramme logique analogue permettant le calcul de la biodégradation sur une verticale d'un piège géologique rempli suivant un deuxième scenario de biodégradation.

**DESCRIPTION DETAILLEE DE LA METHODE**

Le modèle prend en compte une composition de l'huile comprenant huit classes de composés. A chaque classe de composé est associé un bilan stoechiométrique et un facteur de préférence ; de cette façon la simulation permet de suivre l'évolution de composition de l'huile ;

- Il utilise une cinétique de biodégradation avec intégration de mécanismes d'attaque des bactéries au sein du milieu poreux.

**1.1.1. Le modèle**

Comme outil de mise en oeuvre du modèle, on utilise par exemple une plate-forme logicielle connue dite FLUID FOLDER adaptée à la simulation rapide de pièges, des fluides et des phénomènes d'altération : mélange, lessivage, changement de phase, craquage thermique, etc.

Dans ce modèle, on considère un piège (zone poreuse avec une géométrie courbe permettant l'accumulation d'huile) que l'on discrétise par un maillage. Le fluide est biodégradé au sein d'une maille située au niveau de la zone de transition eau/huile du réservoir (Fig. 1a, 1b). Chaque maille est un parallélépipède de base de surface unité qui a pour hauteur l'épaisseur de la zone de transition eau/huile accessible par les bactéries. Au sein de chaque maille (Fig.

2), l'huile est à une saturation variable, depuis la base de la zone qui ne contient que de l'eau jusqu'au sommet de la maille qui contient une quantité résiduelle d'eau, supérieure ou égale à la saturation irréductible de l'ordre de 20%. Pour simplifier le calcul, une saturation globale moyenne est prise en compte dans la maille.

Les grandeurs associées à la maille sont :

- un temps de biodégradation,
- une saturation globale en huile,
- une composition de l'huile (pour le moment en 8 classes de composés),
- une population bactérienne.

**1.1.1.1.** Description du piège

Les grandeurs suivantes sont associées à la zone poreuse :

- La géométrie du piège par exemple la hauteur et la surface dessinant le toit du piège. Elle est connue par imagerie sismique obtenue suite à une campagne de prospection sismique du bassin.
- Les caractéristiques du milieu poreux (porosité, taille des pores, etc). Si le piège géologique étudié n'a pas encore été foré, on peut les évaluer quand on connaît le type des dépôts sédimentaires obtenu suite à des études géologiques régionales préalables. Ce sont généralement des paramètres sensibles influençant la quantité d'hydrocarbures en place. Le modèle permet de tester l'influence de ces paramètres sur l'huile en place. Quand des forages exploratoires ont déjà été forés au travers du piège géologique a déjà été foré, on connaît les caractéristiques du milieu poreux par le résultat de diagraphies ou de carottage.
- Le flux d'hydrocarbures et sa variation au cours du temps. Il est connu ou estimé en faisant « tourner » un outil connu de modélisation de bassin tel que TEMIS par exemple.
- L'épaisseur de la zone de transition. Si le piège n'a pas encore été foré, on déduit l'épaisseur de cette zone des relations connues des spécialistes reliant la pression capillaire et l'épaisseur de la zone de transition.
- La composition de l'huile. Si l'on ne dispose pas de mesures in situ, on utilise comme composition celle que l'on obtient en faisant tourner un outil connu de modélisation compositionnelle de bassin tel que TEMISCOMP par exemple ou bien celle d'une huile appartenant au même système pétrolier mais non biodégradée. On prend en compte une composition avec par exemple huit classes de composés.
- Le flux en accepteurs d'électrons et sa variation au cours du temps. Le flux d'eau dans le piège est également le résultat d'une modélisation au moyen d'un modèle de bassin. Si le piège étudié n'a pas encore été foré, la composition de l'eau que l'on considère est celle d'une zone connue du bassin sédimentaire où le piège se trouve. La composition de l'eau peut aussi être obtenue en faisant tourner un outil connu de simulation de diagénèse tel que DIAPHORE par exemple. Quand le piège a déjà été foré, la composition de l'eau que l'on retient est à priori la composition actuelle de l'aquifère.
- Des informations générales concernant l'adaptation des bactéries au milieu correspondant au réservoir : taille moyenne d'une bactérie, rythme de consommation des accepteurs d'électrons, facteurs de préférences absolus pour les diverses classes de molécules. Si le piège n'a pas encore été foré, on se réfère à des résultats connus obtenus en laboratoire. Quand le piège a déjà été foré jusqu'à l'aquifère, on effectue des mesures bactériologiques (très lentes en anaérobie) sur l'eau dans son état actuel.

Dans ce piège deux scénarios géologiques possibles ont été étudiés pour modéliser la biodégradation :

*1.1.1.1.1.* Les équations ci-après régissant ces grandeurs dans la maille sont :

a) les équations de bilan de matière qui régissent la maille :

- l'équation bilan de l'huile tenant compte de l'huile qui alimente la maille par convection et de celle qui est éliminée par la réaction de biodégradation ;
- l'équation bilan de l'accepteur d'électrons qui alimente la maille par diffusion et convection et est éliminé par biodégradation ; la biodégradation est pilotée par le rythme de consommation de la population bactérienne ;
- l'équation régissant la population bactérienne ; cette population est ajustée en fonction de l'interface eau/huile, du volume disponible et de la quantité d'accepteurs d'électrons.

$$\frac{dC_1}{dt} = \left(\frac{dC_1}{dt}\right)_{convection} - X_{rel_i}(C_i).stoechio(i).\frac{d[Acc]}{dt}, \qquad (2)$$

$$\frac{d[Acc]}{dt} = \left(\frac{d[Acc]}{dt}\right)_{diffusion} + \left(\frac{d[Acc]}{dt}\right)_{convection} - Fa\left(\frac{dB}{dt}\right), \qquad (3)$$

$$\frac{dB}{dt} = f\left(\sum_j C_j\right) - Fb\left(\frac{d[Acc]}{dt}\right) \qquad (4)$$

Dans ces équations,

- [Acc] est la concentration massique en accepteurs d'électrons ;
- [$C_i$], la concentration massique en l'hydrocarbure i ;
- B, la population bactérienne (en unités/ml) ;
- stoechio(i), le coefficient stoechiométrique de biodégradation dans la réaction impliquant l'hydrocarbure i :

$$\text{Hydrocarbure i + stoechio(i) [Acc]-> produits}$$

- Xrel_i, le facteur de préférence relatif de l'hydrocarbure i.

$Fa\left(\frac{dB}{dt}\right) = Kcin\frac{dB}{dt}$, fonction cinétique du premier ordre dépendant de la population bactérienne et correspondant à sa respiration.

$$Fb\left(\frac{d[Acc]}{dt}\right) = \begin{cases} 0 & \text{s'il.y.a.plus.d'accepteurs.d'électrons.que.les.besoins.des.bactéries} \\ \frac{1}{Kcin}\frac{d[Acc]}{dt} & \text{sinon} \end{cases}$$

$f\left(\sum_{j} C_j\right)$ représente le nombre de bactéries nécessaires pour couvrir l'interface d'une monocouche dans la limite de l'espace disponible (il doit rester au moins 20% de volume poreux libre).

*1.1.1.1.2. Les classes de composés et leurs coefficients de préférence.*

Les classes de composés choisies pour représenter l'huile sont déduites des échelles d'avancement de biodégradation de Peters et Moldowan correspondent à l'état actuel des connaissances sur les facteurs de préférence, telles que définies dans la publication suivante par exemple :

- Peters K. E. and Moldowan J. M., "The Biomarker Guide", eds Printice hall, 1993.

Ce sont les suivantes par ordre de préférence d'attaque :

1- C6-
2- N-paraffines C6-C15.
3- Isoparaffines C6-C15.
4- Isoprénoïdes C6-C15.
5- Naphtènes C6-C15.
6- Aromatiques C6-C15.
7- Saturés C15+.
8- Aromatiques C15+.

Chaque classe de composé se voit attribuer un coefficient de préférence absolu et relatif.

*Le coefficient de préférence absolu* est la quantité (par rapport à l'huile totale) de cette classe de composé consommée si on place les bactéries en situation où elles ont un égal accès à chaque classe de composé. Ce coefficient exprime une attirance dans l'absolu des bactéries envers les différentes classes de composés.

*Le coefficient de préférence relatif* d'une classe de composé i est déduite du

$$X_{rel_i} = \frac{X_{abs_i} \times [C_i]}{nombre_de_composés}$$ , Xabs_i étant le facteur de préférence absolue à l'égard de l'hydrocarbure i

coefficient absolu pondéré de la teneur en la classe de composé.

$$X_{rel_i} = \frac{X_{abs_i} . [C_i]}{nombre_de_classe_de_composés}$$ , où $X_{abs_i}$ est le facteur de préférence absolue à l'égard de l'hydrocarbure i.

*1.1.1.1.3. Cinétique biochimique.*

En première hypothèse, les bactéries ne fonctionnent qu'en respiration. Cela signifie que l'on néglige dans ce modèle la phase de croissance de la population bactérienne en terme de temps de biodégradation et de quantité de réactifs impliqués dans cette phase de croissance. Le système est ramené à une population bactérienne stable à chaque pas de calcul qui se régénère d'elle-même et qui globalement avec l'environnement extérieur se conduit comme un simple système qui respire.

Comme on l'a déjà dit, le modèle prend en compte également d'une cinétique de biodégradation qui est fonction de la population bactérienne et pas seulement des réactifs comme le veut la loi de Monod notamment. La cinétique biochimique ne dépend plus seulement de la quantité en réactifs apportés par le cadre géologique. La population bactérienne influe sur la quantité de réactifs mis en jeu.

Pour tenir compte de cette cinétique, on calcule la quantité d'accepteurs d'électrons impliqués dans la biodégradation qui est fonction de la population bactérienne. Si la quantité importée géologiquement est surabondante par rapport à la population bactérienne maximale, seule une partie de cette quantité est effectivement consommée ; sinon on considère qu'elle est entièrement consommée. On applique alors une loi de type loi du premier ordre.

*Calcul de la population bactérienne*

Comme cela a déjà été dit, les bactéries tendent à s'associer en flocs biologiques et à augmenter la superficie de l'interface eau/huile. On fait l'hypothèse simplificatrice pour le modèle que ces mécanismes sont limités par le volume disponible, la porosité allant en diminuant avec la profondeur. On considère que la population bactérienne occupe, à l'échelle des gouttelettes dans le milieu poreux, l'interface eau/huile d'une monocouche.

Pour calculer la population bactérienne, le milieu poreux est représenté par un milieu équivalent plus simple géométriquement (Fig. 3a, 3b). Chaque interstice poreux est ramené à une cavité sphérique. Au sein de chaque interstice de la porosité, l'huile à la saturation courante est rassemblée en une goutte sphérique. On calcule le nombre maximal $N_b$ de bactéries pouvant couvrir cette surface, sachant que la surface spécifique $A_b$ de chaque bactérie pouvant couvrir la gouttelette vaut :

$$N_b = \text{Interface} / A_b \text{ où } A_b = \pi . R_b^2 \text{, avec } R_b \text{ : rayon moyen d'une bactérie.}$$

Ce nombre de bactéries obtenu est ensuite à ajuster selon deux critères :

*1) L'espace poreux libre restant (une fois comptabilisé le volume occupé par l'huile et la population bactérienne) doit être supérieur à 20%.*

Si l'espace poreux libre est inférieur à 20%, alors la population est ajustée de telle façon que l'espace poreux libre vaille 20% (20% est une valeur arbitraire "raisonnable" laissant une certaine mobilité aux bactéries). Dans ce cas cela signifie que la population bactérienne n'est pas en nombre suffisant pour couvrir toute l'interface. Une partie des molécules est alors dissoute dans l'aquifère et les bactéries auront aussi intérêt à occupée l'aquifère pour capter ces molécules.

La figure 4 illustre un exemple d'application où l'on a déterminé comme indiqué ci-dessus, la population bactérienne pour des bactéries de rayon moyen 1 micron. La population est calculée avec une porosité variable et une saturation en huile également variable. Les droites pentues correspondent à une population qui n'a pas besoin de correction, l'espace libre étant supérieur à 20%. Les droite horizontales indiquent une population ajustée pour obtenir 20% de porosité. On voit qu'il faut une taille de pore minimale de 100 microns environ et une saturation en huile assez faible pour espérer avoir un recouvrement complet de l'interface.

2) *Les besoins des bactéries en accepteurs d'électrons au cours de la durée associée à la maille doivent être inférieurs ou égaux à la quantité d'accepteur d'électrons apportée.*

Si la quantité d'accepteurs d'électrons apportée par l'aquifère est inférieure à la quantité qui est nécessaire pour la survie de l'ensemble de la population compatible avec la quantité d'hydrocarbures, la population bactérienne va se réduire en un temps très réduit (une seule génération de quelques heures doit suffire en pratique) pour venir s'ajuster à la quantité d'accepteurs d'électrons disponible. Dans ce cas, et ce cas seulement, cela signifie qu'au final on revient à un système dont la cinétique est contrôlée par l'apport en accepteurs d'électrons, et on peut écrire les bilans équationnels directement avec une loi de type Monod bien connue des spécialistes reliant la vitesse de croissance bactérienne varie avec la quantité de biomasse présente.

Ainsi, ce modèle développe une nouvelle approche qui tient compte des stratégies d'attaque de l'huile par les bactéries et de la population bactérienne présente pour contrôler la cinétique réactionnelle.

*Calcul de la quantité d'huile et d'accepteurs d'électrons apportés par convection.*

Dans le modèle, la convection est un phénomène à flux constant pour une maille du maillage vertical mais peut être variable lors du remplissage de la maille suivante située en dessous. Du fait de la géométrie du piège, si le flux reste contant lors du remplissage de plusieurs mailles consécutives, la quantité d'huile et d'accepteurs d'électrons dans les mailles seront variables.

La grandeur utilisée est pour l'huile le volume annuel arrivant dans le piège. Cette grandeur est à diviser par le nombre de mailles que l'on peut disposer latéralement au sein de la zone de transition, quantité qui est variable en fonction de la géométrie du piège, pour obtenir la quantité d'huile remplissant une seule maille à chaque unité de temps.

Dans les simulations réalisées, la valeur du cas de référence prise pour ce flux est de 1,4 l/an ; ceci permet un remplissage du piège gaussien de hauteur 100 m et de largeur 2000 m en 100 000 ans. Pour les accepteurs d'électrons, la grandeur utilisée est le masse d'accepteurs d'électrons qu'un ml d'eau voit défiler en un an dans l'aquifère.

Dans les simulations réalisées pour le cas type, on a considéré une saturation en accepteurs d'électrons de 25 ppm/ml d'eau. Si l'on considère uniquement de l'oxygène de masse molaire 16g/mol, cela fait pour un aquifère se déplaçant de 1 cm/an un flux de 2 mg/ml d'eau/an.

*Calcul de la quantité d'accepteurs d'électrons diffusés*

La diffusion s'opère verticalement depuis la colonne d'eau considérée comme un milieu infini de concentration constante en accepteur d'électrons jusqu'à la zone de transition. La diffusion crée un gradient de concentration au sein de la zone de transition. Pour simplifier, on calcule une concentration moyenne pour toute la zone de transition.

Un tel système est régi par la loi de Fick ; l'équation bilan est donc : $\frac{\partial C}{\partial t}(z,t) = K\frac{2\Phi}{3-\Phi}\frac{\partial^2 C}{\partial z^2}(z,t)$ où K est le coefficient de diffusion, et $\frac{2\Phi}{3-\Phi}$ représente la tortuosité (porosité).

La résolution de cette équation donne :

$$\frac{C(z,t)-CO}{C(z,0)-CO} = \mathrm{erf}\frac{z}{2\left(\frac{2\phi}{3-\phi}Kt\right)^{1/2}}$$

$$\frac{C(z,t)-CO}{C(z,0)-CO} = erf\frac{z}{2\left(\frac{2\Phi}{3-\Phi}Kt\right)^{1/2}} \quad \text{avec} \quad erf(u) = \frac{2}{\sqrt{u}}\int_0^u e^{-x^2}dx$$

Le calcul de la valeur moyenne de la concentration C donne :

$$\overline{C} = \frac{1}{L}\int_0^L Cz,t)dx$$

## Scenarios de remplissage du réservoir

Deux scénarios sont possibles selon que l'accumulation d'huile dans le piège s'est faite dans des conditions favorables (notamment une températures compatible) ou non aux phénomènes de biodégradation.

*Scénario 1*

Dans le premier scénario, illustré par la Fig. 1a, la biodégradation se produit très tôt, dès le début de la phase de remplissage du piège par l'huile, du fait que la température à la profondeur d'enfouissement du piège se prête à une action des bactéries. La zone de transition est alimentée par de l'huile non biodégradée à flux régulier et par de l'eau chargée en accepteurs d'électrons. Le front de biodégradation se déplace donc vers le bas au fur et à mesure du remplissage. La géométrie du piège offre aux bactéries un temps de remplissage variable, de plus en plus long si le piège s'évase. Avec un flux constant en hydrocarbures, un gradient de biodégradation va donc se créer dans la colonne d'huile. L'huile qui migre jusqu'au piège vient alimenter la zone de transition et l'ensemble du champ. Un mélange entre de l'huile dégradée et non dégradée s'opère en permanence. Comme on le voit sur la Fig.6, la proportion d'huiles lourdes croît avec la profondeur

L'organigramme de la Fig.8, permettant la mise en oeuvre de la méthode dans ses différentes étapes, suivant le scénario 1 sont les suivantes :

- 1 - Introduction des données dont à besoin le modèle.

- 2 - Calcul en fonction de la géométrie du piège et de la l'épaisseur de la zone de transition du nombre de mailles correspondant à la hauteur fermée du piège. Cette valeur est la hauteur de la zone fermée divisée par la hauteur de la zone de transition. Un flux entrant d'hydrocarbures est calculé pour chacune des mailles, ce flux correspond au flux total entrant dans le gisement divisé par le volume total de la zone de transition à l'échelle du champ (hauteur de la zone de transition * surface du gisement à la profondeur de la maille).

- 3 - Départ du calcul de biodégradation dans la première maille, ce calcul est une boucle qui tourne jusqu'à ce que la porosité dans la maille soit remplit à 80% par de l'huile, ce calcul prend en compte les valeurs de flux en hydrocarbures "frais" et accepteurs d'électrons entrant dans la maille ainsi que la destruction d'une partie de ces hydrocarbures par les bactéries.

- 4 - Passage à la maille suivante située immédiatement en dessous et reprise du calcul de biodégradation dans cette nouvelle maille; et ce jusqu'à la dernière maille.

- Le sens de progression de maille à la maille voisine s'effectue ici de haut en bas.

*Scénario 2*

Dans le second scénario illustré par la Fig.1b, en raison d'un trop grand enfouissement du piège et donc d'une température trop élevée, défavorable à l'activité bactérienne, la biodégradation n'a commencé à se produire qu'à un stade tardif de remplissage du piège par de l'huile non biodégradée. De ce fait, les bactéries attaquent l'huile depuis la base du champ dans la zone de transition alimentée par l'aquifère chargée en accepteurs d'électrons. La consommation d'huile réduit son volume dans la zone de transition, la saturation en huile diminue donc dans cette zone et, par équilibre lié aux pressions capillaires, la zone de transition est translatée vers le haut permettant à l'eau et aux bactéries de s'infiltrer lentement dans le champ.
Il en résulte que la biodégradation n'a pas pu se propager très en profondeur vers le haut du piège, si bien que une proportion souvent très importante de l'huile accumulée n'a pas été dégradée. C'est le cas le plus favorable recherché par les opérateurs. La méthode proposée leur permet de sélectionner les conditions d'exploitation du réservoir.
L'organigramme de la Fig. 9, permet la mise en oeuvre de la méthode dans ses différentes étapes, suivant le scénario 2. Il diffère de celui de la Fig. 8 essentiellement par le sens de progression de maille en maille qui est ici de bas en haut, ce qui change les enchaînements.
Le choix du scénario 1 ou du scénario 2 pour traiter le piège prospecté nécessite de connaître les conditions de sa formation et ses déplacements en profondeur qui conditionnent la température de remplissage. On fait ce choix d'après les résultats d'une simulation effectuée à l'aide d'un modèle de bassin tel que Témis 2 ou 3D ou d'un modèle 1D tel que Genex.
Connaissant la composition de l'huile biodégradée, en utilisant un module de calcul thermodynamique gaz-huile classique, on peut calculer la densité de l'huile et en déduire le degré API des huiles dans le piège en fonction de la profondeur.

**Validation de la méthode sur des composants réels**

La figure 6 montre les résultats d'une application du modèle à un piège de forme Gaussien et la figure 7 présente un cas d'application réel à un champ pétrolier.
Le fluide de départ est une huile provenant d'un champ d'Amérique du Sud. Ce champ est biodégradé et une série d'échantillons de degré de biodégradation variables ont été fournis. Au sein de ce champ en outre la biodégradation s'est effectuée par à-coups successifs, le système étant alimenté régulièrement par des pulses d'huile fraîche, tantôt dégradées, tantôt non. L'huile non biodégradée vient se mélanger avec l'huile dégradée antérieurement.
L'exercice a consisté à caler les différents coefficients de préférence et stoechiométriques. Au sein de chaque maille, l'huile dégradée a été mélangée avec de l'huile non dégradée à taux de mélange constant de 25% pour reproduire les épisodes d'huile sans dégradation. Cette approche a permis de reproduire les deux échantillons biodégradés pris en compte comme l'indique la figure 7.
On a décrit isolément le modèle de biodégradation en utilisant certaines données obtenues en amont au moyen d'un modèle de bassin. Il est bien évident que l'outil logiciel permettant la mise en oeuvre de la méthode pourra être avantageusement intégré comme module de complément dans un outil de modélisation de bassin de manière à bénéficier directement des résultats de modélisation qu'il peut fournir.

## Revendications

1. Méthode pour modéliser la biodégradation progressive d'hydrocarbures piégés dans un réservoir pétrolier ou piège étudié, par action d'une population bactérienne dans un aquifère, à partir de données relatives au réservoir étudié, portant sur la forme et la hauteur du réservoir, les caractéristiques physiques du milieu poreux, l'épaisseur de la zone de transition entre les hydrocarbures et l'eau, la composition des hydrocarbures, du flux d'accepteurs d'électrons rentrant dans le réservoir et de données sur la population bactérienne dans l'aquifère, dans le but de déterminer les conditions d'exploitation du réservoir, **caractérisée en ce qu'**elle comporte :

   - la discrétisation du réservoir par un maillage dont chaque maille a pour hauteur l'épaisseur de la zone de transition ; et
   - la détermination de la variation sur la hauteur du dit réservoir, de la proportion en fractions lourdes des hydrocarbures sous l'effet de la biodégradation par ajustement progressif au sein de chaque maille de la population bactérienne à la quantité d'hydrocarbures disponible, à l'espace poreux disponible, à la quantité d'accepteurs d'électrons présente dans le réservoir et des capacités de dégradation de la dite population.

2. Méthode selon la revendication 1, **caractérisée en ce que** l'on détermine au préalable le taux de remplissage initial du réservoir en hydrocarbures quand les conditions et notamment la température régnant dans le réservoir se prêtent à la biodégradation.

## Claims

1. Method for modelling progressive biodegradation of hydrocarbons trapped in a petroleum reservoir or trap under study, by action of a bacterial population in an aquifer, from data relating to the reservoir under study concerning the form and height of the reservoir, the physical characteristics of the porous medium, the thickness of the transition zone between the hydrocarbons and the water, the composition of the hydrocarbons, the flow of electron acceptors entering the reservoir and data relating to the bacterial population in the aquifer, in order to determine the development conditions of the reservoir, **characterised by** the fact that it includes:

   - quantization of the reservoir by gridding in which each cell has as its height the thickness of the transition zone; and
   - determination of the variation over the height of the said reservoir of the proportion of heavy fractions of the hydrocarbons under the effect of biodegradation by iterative adjustment in each cell of the bacterial population to the available quantity of hydrocarbons, to the available porous space, to the quantity of electron acceptors present in the reservoir and of the degradation capacity of the said population.

2. Method as described in claim 1, **characterised by** the fact that the initial rate of filling of the reservoir with hydrocarbons when the conditions and in particular the temperature in the reservoir lend themselves to biodegradation is predetermined.

## Patentansprüche

1. Verfahren zur Modellierung der progressiven Biodegradation von Kohlenwasserstoffen, die in einem Erdöllager oder untersuchten Reservoir gefangen sind, durch das Einwirken einer Bakterienpopulation in einer wasserführenden Schicht, ausgehend von Daten, die das untersuchte Reservoir betreffen, gerichtet auf die Form und die Höhe des Lagers, die physikalischen Eigenschaften des porösen Mediums, die Dichte der Übergangszone zwischen den Kohlenwasserstoffen und dem Wasser, der Zusammensetzung der Kohlenwasserstoffe der Elektronenakzeptorflüsse, die in das Lager eintreten, und Daten über die Bakterienpopulation in der wasserführenden Schicht, mit dem Ziel, die Abbaubedingungen des Lagers zu bestimmen, **dadurch gekennzeichnet, dass** sie umfassen:

   - die Diskretisierung des Lagers durch ein Netz bei dem jede Masche die Dicke der Übergangszone als Höhe hat; und
   - die Bestimmung der Variation der Höhe des Lagers, des Anteils an schweren Fraktionen der Kohlenwasserstoffe unter der Wirkung der Biodegradation durch progressive Anpassung im Rahmen jeder Masche der Bakterienpopulation an die verfügbare Menge an Kohlenwasserstoffen, an den verfügbaren porösen Raum, an die Menge der Elektronenakzeptoren, die im Lager vorhanden sind, und an die Degradationskapazitäten der Population.

2. Verfahren nach Anspruch 1, **dadurch gekennzeichnet, dass** der Anfangsfüllungsgrad des Lagers mit Kohlenwasserstoffen im Voraus bestimmt wird, wenn sich die Bedingungen und insbesondere die im Lager herrschende Temperatur für die Biodegradation eignen.

FIG.1A

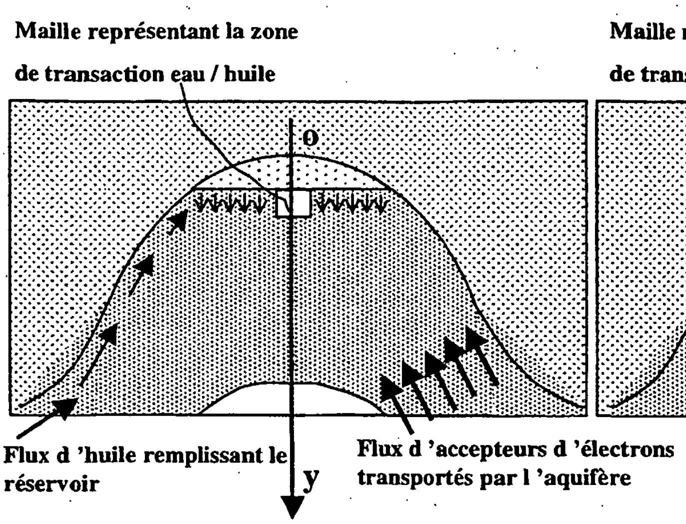


FIG.1B

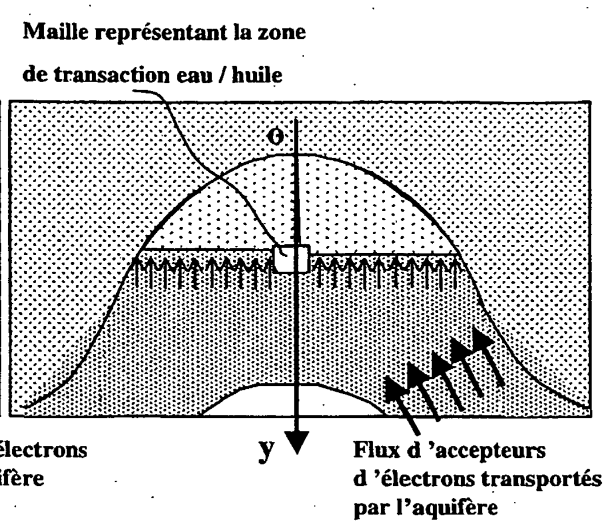


FIG.2

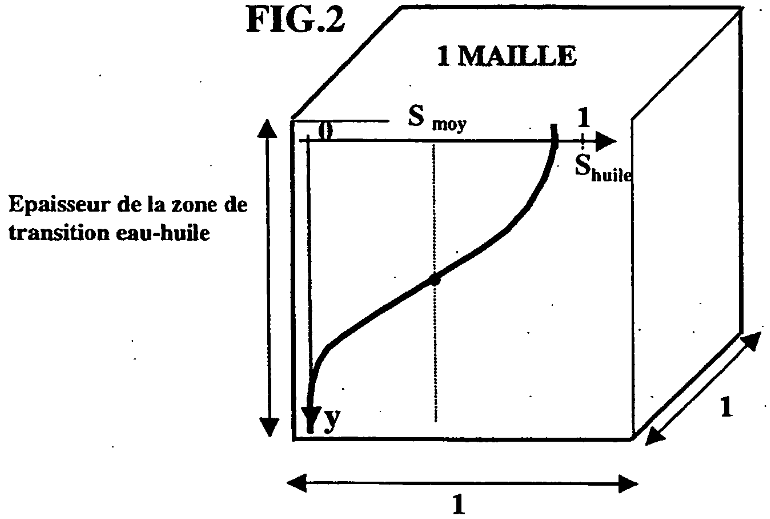


FIG.3A

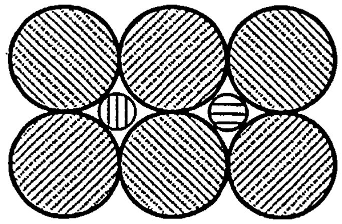

FIG.3B

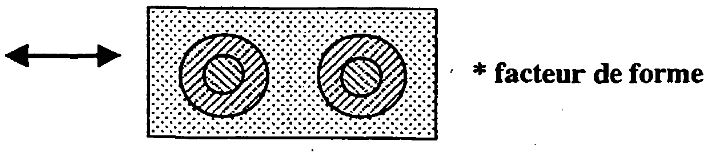

FIG.4

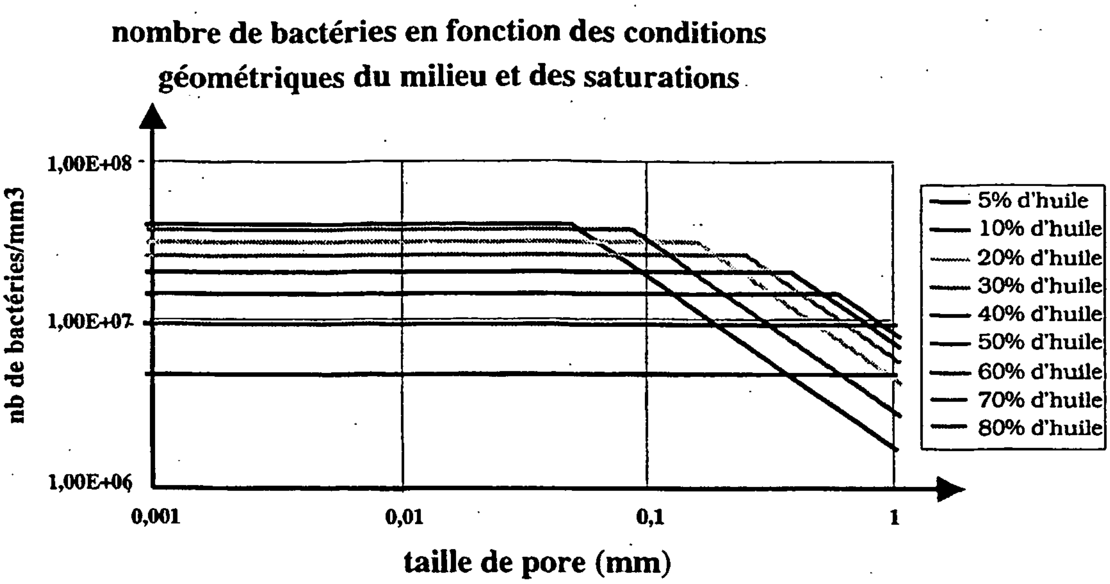
nombre de bactéries en fonction des conditions géométriques du milieu et des saturations
nb de bactéries/mm3
1,00E+08
1,00E+07
1,00E+06
0,001
0,01
0,1
1
taille de pore (mm)
5% d'huile
10% d'huile
20% d'huile
30% d'huile
40% d'huile
50% d'huile
60% d'huile
70% d'huile
80% d'huile

FIG.5

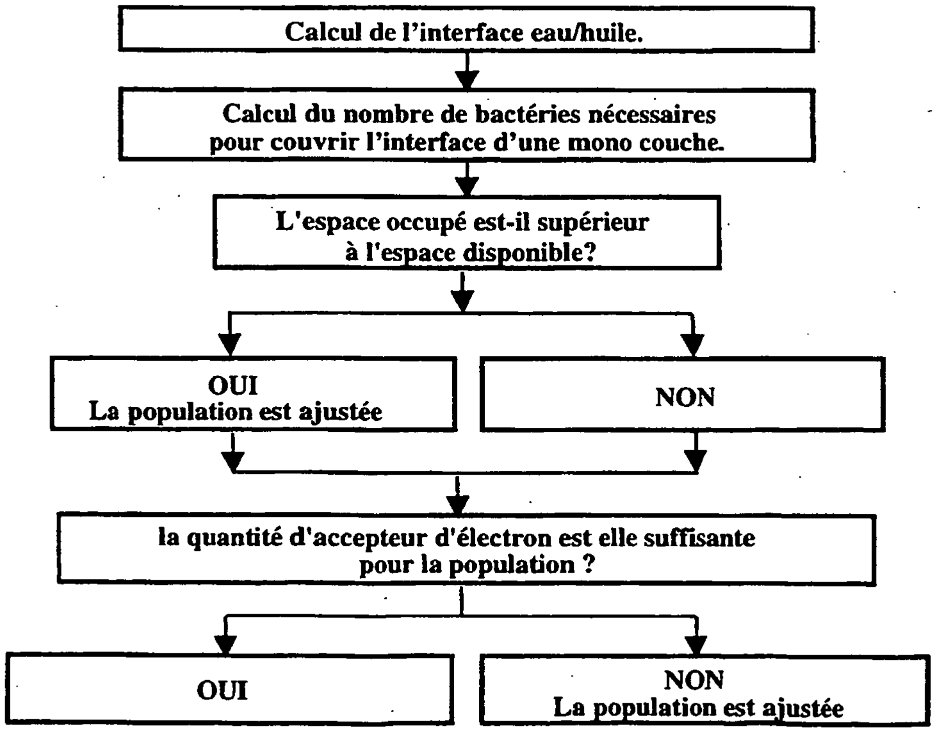
Calcul de l'interface eau/huile.
Calcul du nombre de bactéries nécessaires pour couvrir l'interface d'une mono couche.
L'espace occupé est-il supérieur à l'espace disponible?
OUI La population est ajustée
NON
la quantité d'accepteur d'électron est elle suffisante pour la population ?
OUI
NON La population est ajustée

FIG.6

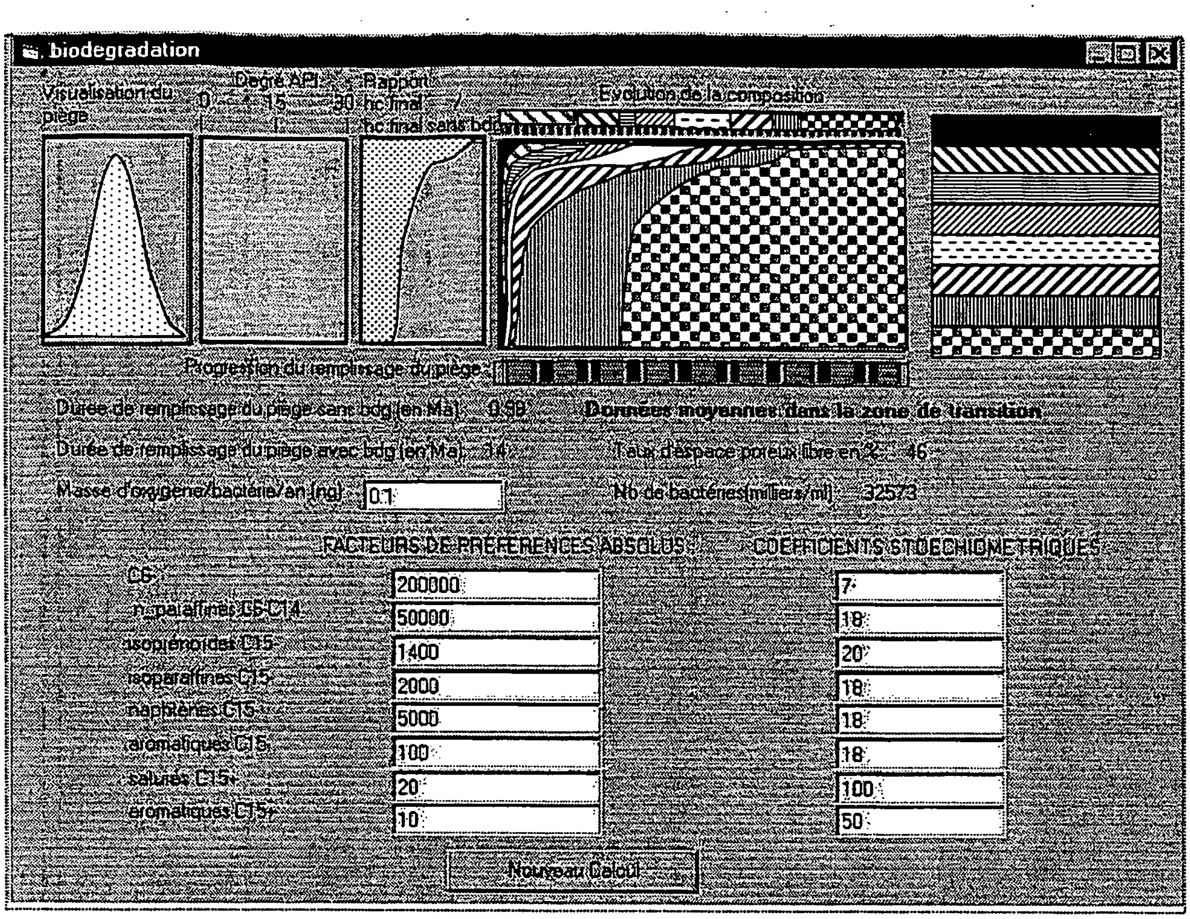
biodegradation
Visualisation du piège
Degré API
0
15
30
Rapport
hc final /
hc final sans bdg
Evolution de la composition
Progression du remplissage du piège
Durée de remplissage du piège sans bdg (en Ma): 0.98
Durée de remplissage du piège avec bdg (en Ma): 14
Masse d'oxygène/bactérie/an (ng)
0.1
Données moyennes dans la zone de transition
Taux d'espace poreux libre en %: 46
Nb de bactéries(milliers/ml): 32573
FACTEURS DE PREFERENCES ABSOLUS
COEFFICIENTS STOECHIOMETRIQUES
C6-
n_paraffines C6-C14
isoprénoïdes C15-
isoparaffines C15-
naphtènes C15-
aromatiques C15-
saturés C15+
aromatiques C15+
200000
50000
1400
2000
5000
100
20
10
7
18
20
18
18
18
100
50
Nouveau Calcul

FIG.7

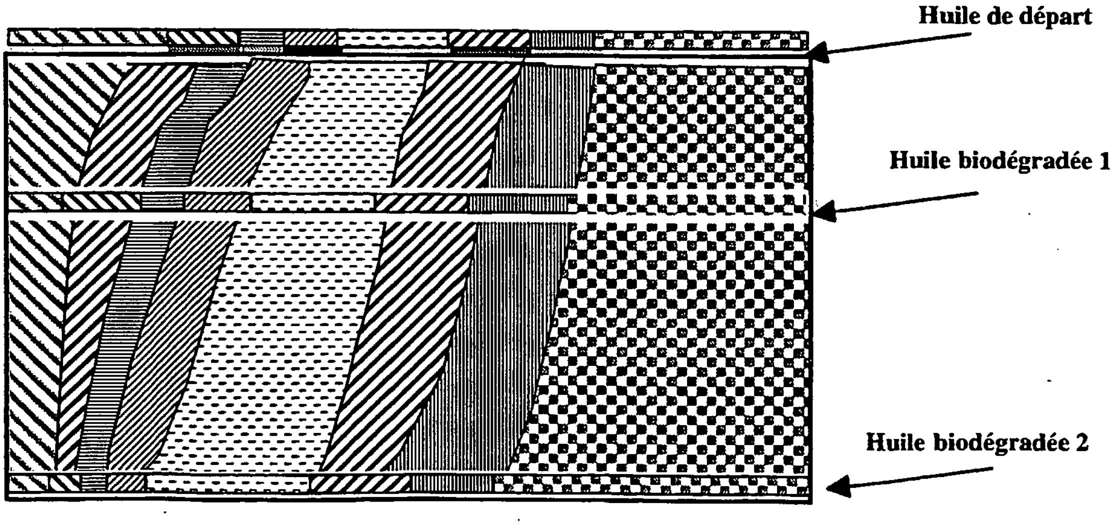
Huile de départ
Huile biodégradée 1
Huile biodégradée 2

FIG.8

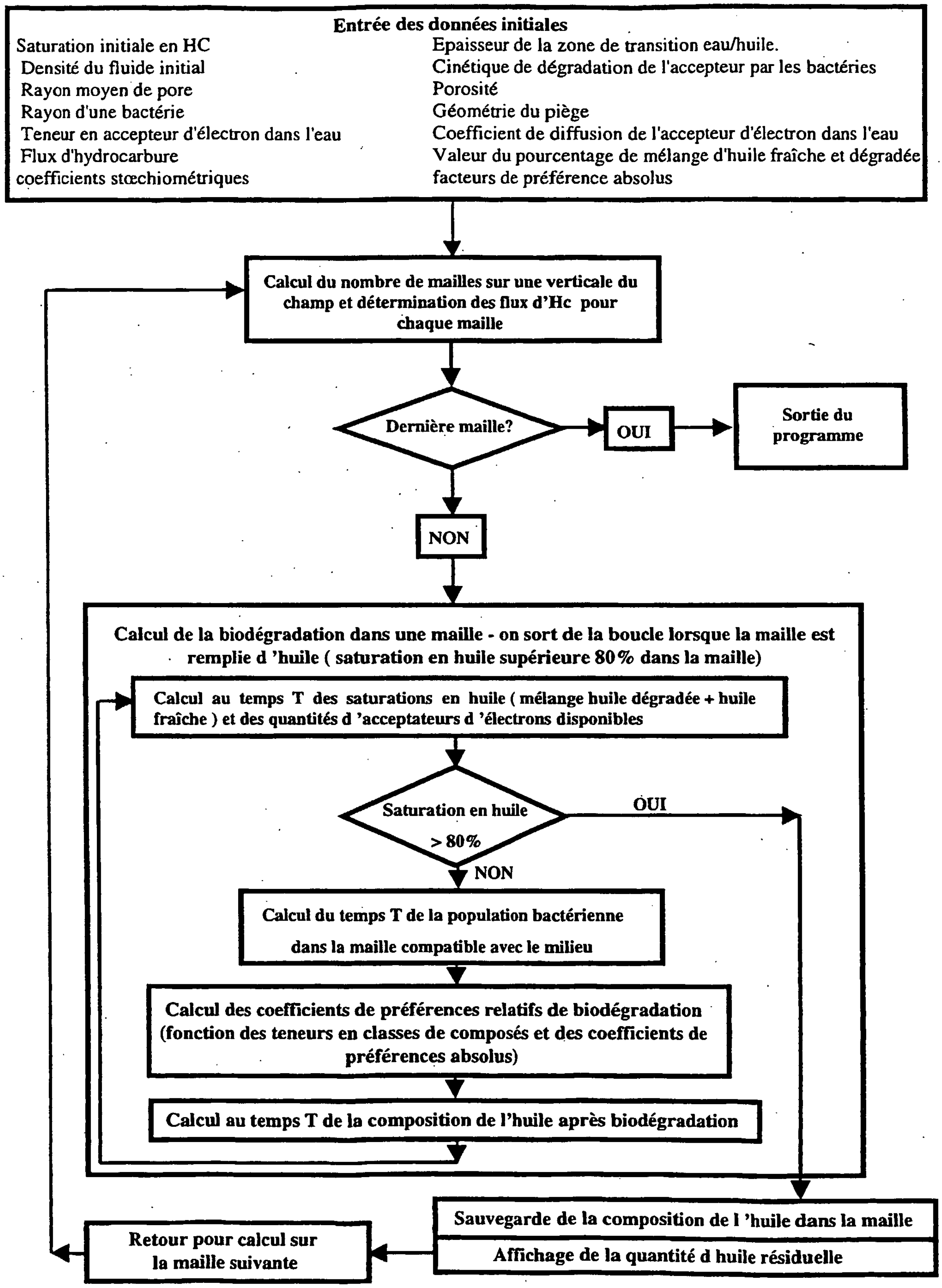
Entrée des données initiales
Saturation initiale en HC
Densité du fluide initial
Rayon moyen de pore
Rayon d'une bactérie
Teneur en accepteur d'électron dans l'eau
Flux d'hydrocarbure
coefficients stœchiométriques
Epaisseur de la zone de transition eau/huile.
Cinétique de dégradation de l'accepteur par les bactéries
Porosité
Géométrie du piège
Coefficient de diffusion de l'accepteur d'électron dans l'eau
Valeur du pourcentage de mélange d'huile fraîche et dégradée
facteurs de préférence absolus
Calcul du nombre de mailles sur une verticale du champ et détermination des flux d'Hc pour chaque maille
Dernière maille?
OUI
Sortie du programme
NON
Calcul de la biodégradation dans une maille - on sort de la boucle lorsque la maille est remplie d 'huile ( saturation en huile supérieure 80% dans la maille)
Calcul au temps T des saturations en huile ( mélange huile dégradée + huile fraîche ) et des quantités d 'acceptateurs d 'électrons disponibles
Saturation en huile > 80%
OUI
NON
Calcul du temps T de la population bactérienne dans la maille compatible avec le milieu
Calcul des coefficients de préférences relatifs de biodégradation (fonction des teneurs en classes de composés et des coefficients de préférences absolus)
Calcul au temps T de la composition de l'huile après biodégradation
Sauvegarde de la composition de l 'huile dans la maille
Affichage de la quantité d huile résiduelle
Retour pour calcul sur la maille suivante

FIG.9

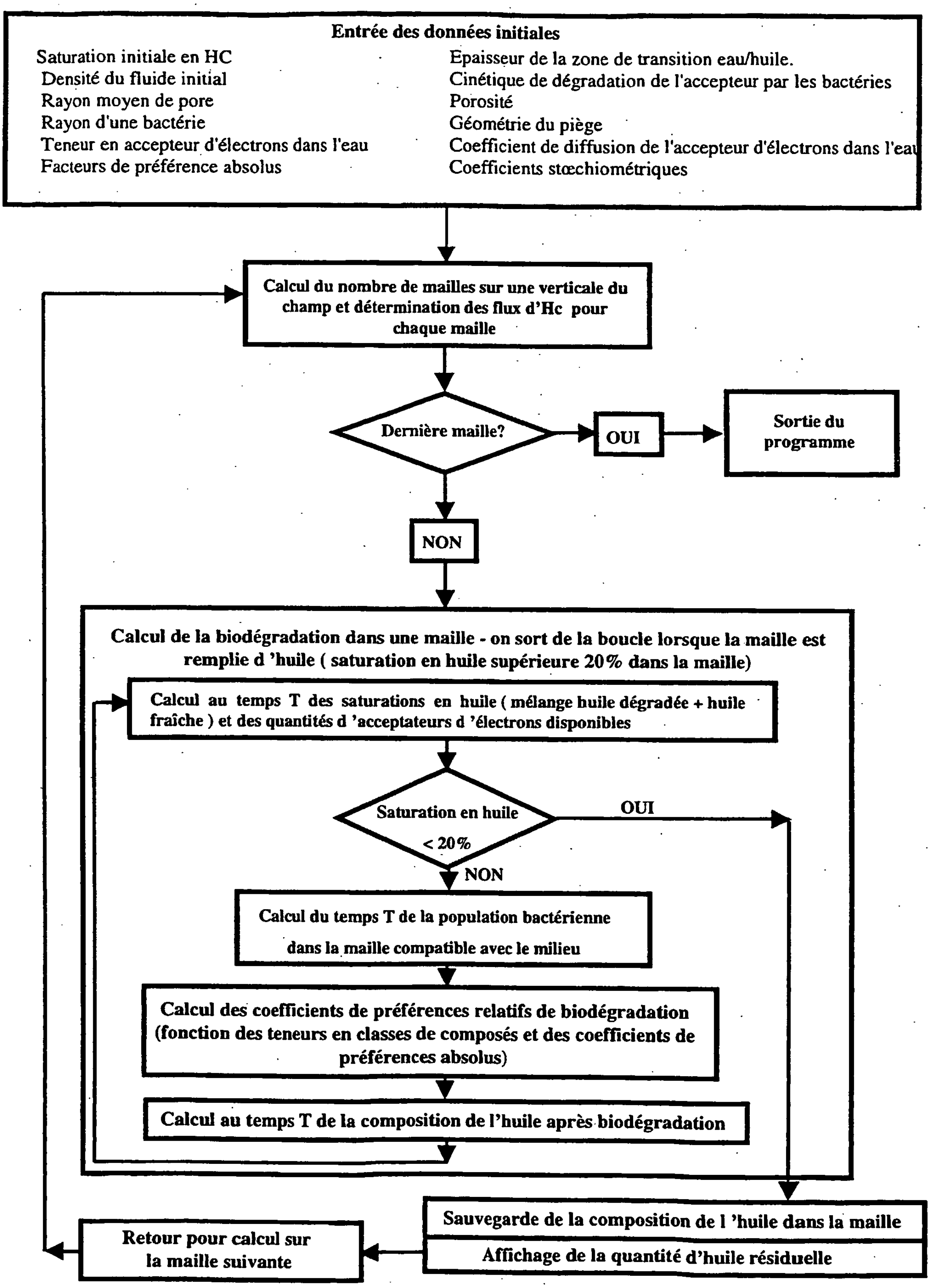
Entrée des données initiales
Saturation initiale en HC
Densité du fluide initial
Rayon moyen de pore
Rayon d'une bactérie
Teneur en accepteur d'électrons dans l'eau
Facteurs de préférence absolus
Epaisseur de la zone de transition eau/huile.
Cinétique de dégradation de l'accepteur par les bactéries
Porosité
Géométrie du piège
Coefficient de diffusion de l'accepteur d'électrons dans l'eau
Coefficients stœchiométriques
Calcul du nombre de mailles sur une verticale du champ et détermination des flux d'Hc pour chaque maille
Dernière maille?
OUI
Sortie du programme
NON
Calcul de la biodégradation dans une maille - on sort de la boucle lorsque la maille est remplie d 'huile ( saturation en huile supérieure 20% dans la maille)
Calcul au temps T des saturations en huile ( mélange huile dégradée + huile fraîche ) et des quantités d 'acceptateurs d 'électrons disponibles
Saturation en huile < 20%
OUI
NON
Calcul du temps T de la population bactérienne dans la maille compatible avec le milieu
Calcul des coefficients de préférences relatifs de biodégradation (fonction des teneurs en classes de composés et des coefficients de préférences absolus)
Calcul au temps T de la composition de l'huile après biodégradation
Sauvegarde de la composition de l 'huile dans la maille
Affichage de la quantité d'huile résiduelle
Retour pour calcul sur la maille suivante